# EUROPEAN PATENT APPLICATION

(11) **EP 3 466 299 A1**
(43) Date of publication of application: **10.04.2019**
(21) Application number: 18788985.2
(22) Date of filing: 21.08.2018
(51) Int. Cl.: A45D 40/26, A45D 40/18, A46B 9/10, A46B 3/00

(54) **MAGNETIC MASCARA**

(30) Priority: 21.08.2017 KR 20170105349
(71) Applicant: COS NINE CO,. LTD., Yangchon-eup Gimpo-si, Gyeonggi-do 10049 (KR)
(72) Inventor: KIM, Hyung-Tae, Seongbuk-gu, Seoul 02704 (KR); HUR, Bum-Chul, Gangseo-gu, Seoul 07517 (KR); PARK, Gun-Ae, Yongsan-gu, Seoul 04378 (KR)
(74) Representative: Zacco GmbH
(86) International application number: PCT/KR2018/009615
(87) International publication number: WO 2019/039842

(57) **Abstract**

The present disclosure provides a novel magnetic mascara which includes a mascara brush stick having a magnet therein, is suitable for the short Asian eyelashes and has excellent curling, long lash and volume effects, and a method for producing the same.

## Description

### TECHNICAL FIELD

The present application claims priority to Korean Patent Application No. 10-2017-0105349 filed on August 21, 2017 in the Republic of Korea, the disclosures of which are incorporated herein by reference.

The present disclosure relates to a magnetic mascara including a magnet. More particularly, the present disclosure provides a novel mascara including a mascara brush stick having a magnet therein so that it may have excellent curling, long lash and volume effects even on the short Asian eyelashes, and a method for producing the same.

### BACKGROUND ART

Mascara is a tool used for eyelash makeup. Eyelash makeup is carried out by curling eyelash up with brush hair to which a mascara solution is applied.

However, the above-mentioned method cannot apply the mascara solution uniformly to each strand of eyelash and may cause partial agglomeration of the mascara solution on the eyelash. In addition, when carrying out eyelash makeup by the above-mentioned method, the eyelash may be localized to one side, a weak curling effect may be provided, and the mascara solution coated on the brush stick portion other than the brush portion may be applied to the portion adjacent to the user's eyes, the user's nose, or the like.

In general, functions required for mascara include a volume effect by which the thickness of each strand of eyelash is increased as compared to its original thickness so that the density of eyelash seems to be increased, a curling effect by which downwardly sagging eyelash is curled up so that the shape of user's eyes seems to be clear, and a long lash effect by which the length of eyelash seems to be increased as compared to its original length. Currently, a large number of ingredients are used to realize each of the functions. The function of mascara depends on the particular ingredients used for the mascara solution. Thus, it is essential to search and apply ingredients having excellent effects. Under these circumstances, many products having each of the volume, curling and long lash effects have been developed, but they cannot provide excellent functions and they have significant disadvantages related with each function undesirably.

The present disclosure refers to many documents throughout the specification and the reference thereof is specified herein. The disclosure of each reference is incorporated herein as a whole by reference so that the present disclosure will be thorough and complete.

### DISCLOSURE

### Technical Problem

The present disclosure is directed to providing a novel magnetic mascara which includes a mascara brush stick having a magnet therein, is suitable for the short Asian eyelashes and has excellent curling, long lash and volume effects, and a method for producing the same.

### Technical Solution

In one aspect of the present disclosure, there is provided a mascara including: a lid used as a knob; a brush stick extended from the inner central portion of the lid; and a container having an inner space for receiving a mascara solution, wherein the free end of the brush stick includes a mascara application head having no brush hair and the inner space of the application head includes a magnet.

### Brush stick Including Magnet and Application Head

The inventors of the present disclosure have focused on the fact that when a mascara application head has brush hair like the appearance of a conventional mascara, loss of magnetic force is high due to the brush hair even in the presence of a magnet in the head, and thus magnetic force cannot be supplied sufficiently and continuously to have an effect on the mascara solution. Therefore, we have manufactured a mascara application head having no brush hair, and have found that use of such a mascara application head could supply stable and uniform magnetic force energy continuously.

The mascara application head according to the present disclosure has no brush hair, and thus the brush stick may be washed and used. Therefore, it is possible to use a single brush stick having a magnet therein to apply mascara solutions having various colors to the eyelash. The washing may be carried out by using water, cloth, tissue, wet tissue, cleansing tissue, cleansing solution, alcohol, or the like, but is not limited thereto.

The mascara application head according to the present disclosure may have a magnet, preferably a stick-type permanent magnet, in the inner space thereof. This allows the application head to react with iron oxide (FeO) contained in the mascara solution, thereby providing curling, long lash and volume-increasing effects to the eyelash by virtue of the magnetic field.

The magnet provided in the application head includes, but is not limited to: an AlNiCo magnet, such as a sintered or cast magnet, semi-hard magnet, AlNiCo-9, AlNiCo-5, or the like; a ferrite magnet, such as an oxide magnet, sintered magnet, Ba-ferrite, Sr-ferrite, or the like; a rare earth metal magnet, such as a sintered magnet, Sm-Co, Nd-Fe-B, Yt-Co, or the like; a rubber magnet, such as a resin magnet, rubber molded magnet, or the like; and a plastic magnet, such as an injection molded magnet.

In addition, the magnet may be an anisotropic magnet magnetized strongly in one direction or isotropic magnet magnetized in multiple directions. When the magnet used herein is an anisotropic magnet, both poles of the magnet may be formed in the longitudinal direction or transverse direction of the brush stick. However, when both poles of the magnet are formed in the longitudinal direction of the brush stick, the magnetic force is supplied intensively only to the end portion of the mascara application head. Thus, it is preferred to use a magnet both poles of which are formed in the transverse direction of the brush stick.

According to an embodiment, the magnet provided in the mascara application head according to the present disclosure may have a diameter of 2-3 mm.

Even when using an application head having no brush hair to minimize loss of magnetic force, a stick-type magnet having a predetermined thickness is required in order to ensure stable and uniform magnetic force energy. In this case, the application head becomes thick, and thus is not suitable for the short Asian eyelashes. Meanwhile, when using a rare earth metal magnet having strong magnetic force, it is possible to reduce the diameter of the application head. However, the product is not cost-efficient due to the high cost of the rare earth metal magnet.

Therefore, the inventors of the present disclosure have solved the above-mentioned problem by manufacturing a brush stick in such a manner that the mascara application head includes a head body portion and a head terminal portion having a smaller diameter as compared to the head body portion, and has a magnet in the inner space of the head body portion but has no magnet in the head terminal portion. Herein, although the head terminal portion has no magnet, the magnetic property derived from the magnet provided in the head body portion may be realized indirectly to the head terminal portion.

According to another embodiment, the head body portion has a cylindrical shape and the head terminal portion may be a tapered cone-shaped tip or a cylindrical tip having a smaller diameter as compared to the head body portion.

The mascara application head including the head body portion and the head terminal portion may be made of a metallic material, preferably stainless steel or steel use stainless (SUS), more preferably ferrite-based stainless steel having magnetization retentivity, or may be made of a resin material including magnetic or magnetizable powder.

The resin material may include, but is not limited to: a synthetic resin material, such as polyethylene terephthalate, polybutylene terephthalate, polyvinyl chloride, acrylonitrile-butadiene-styrene copolymer (ABS), nylon 6, nylon 66, polyethylene fibers, polypropylene fibers, polytetrafluoroethylene, polychlorotrifluoroethylene, tetrafluoroethylene, polyvinylidene fluoride, vinyl acetate, acryl amide, methacrylate or acrylate; or a rubber material, such as natural rubber, styrene-butadiene rubber (SBR), acrylonitrile-butadiene rubber (NBR), polybutadiene rubber (BR), polychloroprene rubber (CR), ethylene-propylene rubber (EPR), ethylene-propylene-diene rubber (EPDM), thiocol, chlorobutyl rubber (CIIR), isobutyl-isoprene rubber (IIR), ethylene vinyl acetate (EVA) or silicone rubber.

The mascara application head may have grooves formed therein, and the grooves may have a quadrangular or triangular shape. Particularly, when the grooves have a triangular shape, the mascara solution may be applied more finely to the eyelash without agglomeration.

According to still another embodiment, the application head body portion may have a diameter of 5-6 mm, the grooves formed in the application head may have a depth of 0.2-0.5 mm, and the grooves may be formed at an interval of 0.5-0.75 mm.

### Packing

According to a preferred embodiment, the inner space for receiving the mascara solution includes a packing at the upper end entrance thereof, wherein the packing has an aperture-like shape.

The packing in the mascara container refers to a part configured to scratch the brush stick in order to wipe out the mascara solution applied excessively on the brush stick and brush so that only a small amount of solution may be applied finely to the eyelash.

In the mascara container according to the present disclosure, the packing may be an elastic material, such as a silicone packing. Preferably, the packing is made of an elastic material in order to wipe the brush stick to the end thereof.

The conventional packing has a round shape to prevent the brush stick from being affected by its introduction into and discharge from the container. However, when the application head has a smaller thickness as compared to the brush stick, the conventional round shaped mascara packing cannot wipe the application head sufficiently.

Thus, in the case of mascara provided with an application head having a smaller diameter as compared to the brush stick, an actuator capable of introducing the application head into the brush head is provided and an additional packing is provided at the entrance of the terminal end of the brush head through which the application head is introduced. In this case, the actuator is allowed to operate to wipe the application head again when the application head is retreated into the brush stick, which makes the overall structure complicate. However, in this case, the remaining mascara solution is inserted into and solidified in the brush stick but cannot be cleaned with ease, which is not hygienically favorable. Moreover, such a complicated structure requires high cost and causes a failure in the actuator undesirably.

The inventors of the present disclosure have solved the above-mentioned problem by using an aperture-like packing at the entrance of the upper end of the inner space for receiving a mascara solution.

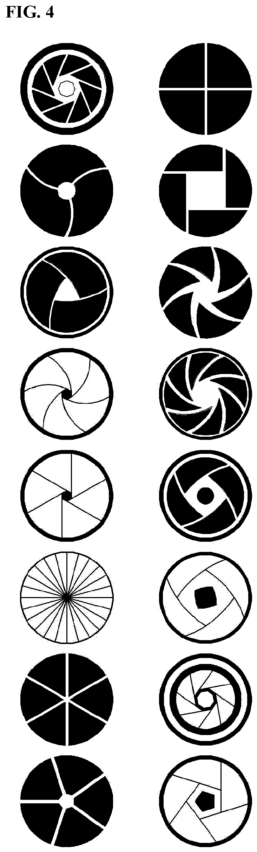

The term 'aperture-like' refers to a shape concentrated to the central point in the absence of external force and then closed, while being opened gradually from the center in all directions like a camera lens upon the application of external force. FIG. 4 illustrates an embodiment of such an aperture-like shape which may be taken by the packing of the mascara according to the present disclosure.

When the packing has an aperture-like shape as mentioned above, the mascara is not affected by the shape of brush stick or head as well as the introduction/discharge of the brush into/from the inner container. In addition, since the thickness of brush or application head has no effect, it is possible to provide a mascara application head capable of trimming the eyelash finely through the use of two or more different diameters.

According to an embodiment, even when the mascara according to the present disclosure has a brush stick having a predetermined thickness, an application head body portion having a smaller thickness as compared to the brush stick and an application head terminal portion having a smaller thickness as compared to the application head body portion, the aperture-like shaped packing provided at the entrance of the upper end of the container can scratch and wipe out all of the brush stick, head body portion and head terminal portion sufficiently.

### Mascara Solution

The mascara solution received in the magnetic mascara container according to the present disclosure may include an iron oxide ingredient. In this case, when the application head having a magnet therein applies the mascara solution to the eyelash while it brushes the eyelash, the iron oxide ingredient is aligned by virtue of the magnetic property of the application head to provide an effect of extending the eyelash.

According to a preferred embodiment, the mascara solution received in the magnetic mascara container according to the present disclosure may include a vegetable wax ingredient drawing the eyelash in addition to the iron oxide ingredient.

In the conventional mascara solution, the vegetable wax has no specific difference in function as compared to animal, mineral or synthetic wax, and is known as an equivalent thereof. However, the inventors of the present disclosure have found that the vegetable wax ingredient having a depilation function and contained in a waxing gel ingredient serves to draw the eyelash, and thus it helps the iron oxide-containing solution to be aligned on the drawn eyelash by the magnetic force when it meets the magnetic mascara, resulting in significant improvement of curling and long lash effects.

Preferably, the vegetable wax ingredient drawing the eyelash may include carnauba wax, rice bran wax or Rhus Succedanea fruit wax.

In general, the vegetable wax contained in mascara includes carnauba wax, candelilla wax, sugar wax, sunflower seed wax and bees wax. However, the conventional vegetable wax, such as candelilla wax, sugar wax, sunflower seed wax and bees wax, has no function of drawing the eyelash. Thus, when using them, it is not possible to accomplish significant curling and long lash effects by virtue of magnetic force and the effect of iron oxide.

According to another preferred embodiment, the vegetable wax ingredient drawing the eyelash is contained in the mascara solution in a large amount. For example, it may be contained in an amount of at least 1% (v/v), at least 2% (v/v), at least 3% (v/v), at least 4% (v/v), at least 5% (v/v), at least 6% (v/v), at least 7% (v/v), at least 8% (v/v), at least 9% (v/v) or at least 10% (v/v).

In addition to the above-mentioned ingredients, the mascara solution according to the present disclosure may further include at least one ingredient selected from the group consisting of an animal, mineral or synthetic wax ingredient, emulsifier, thickener, film-forming agent, pigment, distilled water, moisturizing agent, preservative, and the like.

The animal wax that may be used includes bees wax and lanolin wax, the mineral wax that may be used includes paraffin, ceresin, microcrystalline wax and ozokerite, and the synthetic wax that may be used includes polyethylene wax and wax prepared by the Fischer-Tropsch method.

The emulsifier may be a conventional emulsifier used in the art, the thickener that may be used includes at least one selected from the group consisting of acacia gum, xanthan gum, cargeenin, magnesium aluminum silicate, hydroxyethyl cellulose, hydroxypropyl cellulose, carboxymethyl cellulose and cellulose derivatives.

The film-forming agent that may be used includes at least one selected from the group consisting of polyvinyl alcohol, polyvinyl acetate, polyvinyl acetate, polyvinyl pyrrolidone, ammonium acrylate copolymer, PVP/eicosin copolymer, acrylic/acrylate copolymer, acrylate copolymer, alkyl acrylate copolymer emulsion and hydroxyethyl cellulose dimethyldiallylammonium chloride copolymer (Trade name: Polyquaternium-4).

The pigment that may be used includes an inorganic pigment, organic pigment or pearl pigment. One or more pigments may be used depending on a desired color and color concentration.

The moisturizing agent that may be used includes various kinds of moisturizing agents used currently for a mascara solution, and particular examples thereof include at least one selected from the group consisting of glycerin, 1,4-butylene glycol, propylene glycol, DL-pantenol, sorbitol, glycol distearate and sorbitol hydroxystearate.

The preservative that may be used includes a conventional preservative used currently for a mascara solution, and particular examples thereof include imidazolidinyl urea, diazolidinyl urea, quaternium-15, phenoxyethanol, potassium sorbate, methyl paraoxybenzoate, butyl paraoxybenzoate, propyl paraoxybezoate and ethyl paraoxybenzoate.

In addition, the mascara solution according to the present disclosure may further include a pH modifier, infiltration aid and a stabilizer for formulating a product, and various cosmetic formulating agents, such as a surfactant, conditioning agent, skin emollient, appearance improving agent, fragrance, pigment and a metal ion chelator without departing from the scope of the present disclosure.

In another aspect of the present disclosure, there is also provided a mascara including: (i) one mascara brush stick; and (ii) at least two containers including an inner space opened/closed with a lid. Herein, the free end of the brush stick includes a mascara application head having no brush hair, the inner space of the application head includes a magnet, and the containers individually include at least two mascara solutions having different colors.

As mentioned above, since the mascara application head has no brush hair, it can be washed with the brush stick. Thus, one brush stick having a magnet therein may be used to apply mascara solutions having various colors to the eyelash.

The mascara according to the present disclosure is used by applying it from the root of the eyelash upwardly to the top in a zigzag manner so that the mascara solution containing a large amount of vegetable wax ingredient drawing the eyelash (having a depilation function) may be applied uniformly to each strand of eyelash. Then, iron oxide on the mascara ingredient is attached automatically to the end of the eyelash by the magnetic force so that iron oxide may be aligned in the same direction to extend the eyelash. Therefore, unlike the conventional mascara applying its content to the eyelash by physical force, the mascara according to the present disclosure shows a significant curling effect by the magnetic force and iron oxide applied to the end of the eyelash is linked continuously to provide a clearer long lash effect.

### Advantageous Effects

The mascara according to the present disclosure includes a mascara application head having no brush hair, and thus can be washed and used. In addition, even though the mascara has a magnet therein, it has an effect of finely trimming even the short Asian eyelashes.

### DESCRIPTION OF DRAWINGS

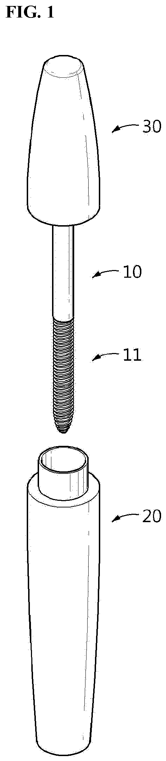

FIG. 1 illustrates the mascara according to an embodiment of the present disclosure.

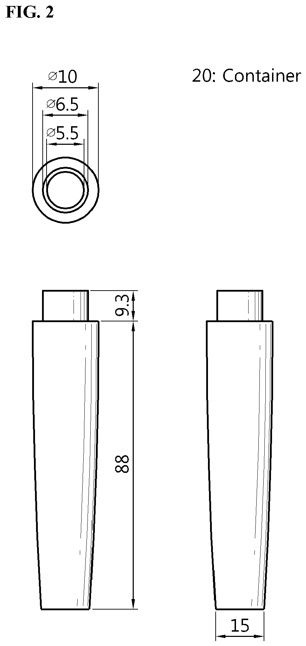

FIG. 2 illustrates the appearance of the mascara container 20 according to an embodiment of the present disclosure.

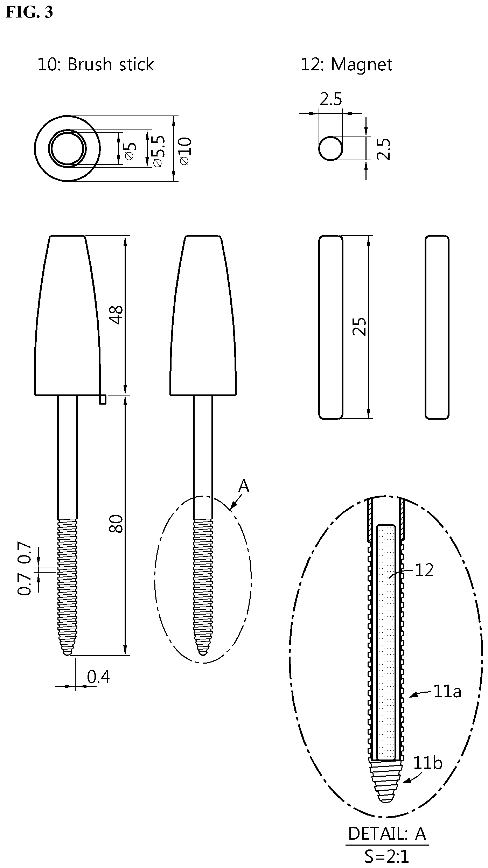

FIG. 3 illustrates the mascara brush stick 10 and the magnet 12 provided therein according to an embodiment of the present disclosure, wherein the head terminal portion has a shape of tapered cone-like tip.
FIG. 4 illustrates the aperture shape which may be taken by the packing of the mascara according to an embodiment of the present disclosure.

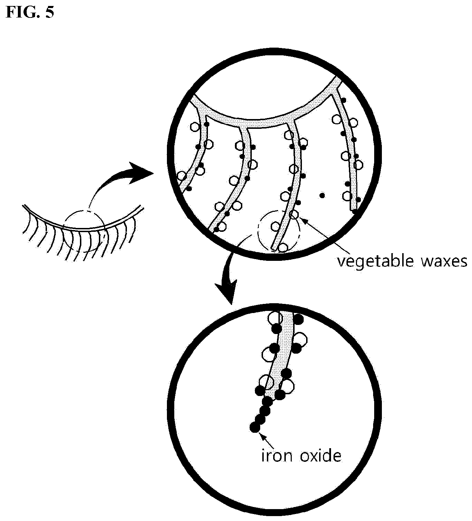

FIG. 5 illustrates the principle of the effect of the mascara solution including iron oxide in combination with a vegetable wax drawing the eyelash under the magnetic force according to an embodiment of the present disclosure.

### MODE FOR DISCLOSURE

Hereinafter, the present disclosure will be described in detail with reference to examples. However, it should be understood that the following examples are for illustrative purposes only and it is apparent to those skilled in the art that the scope of the present disclosure is not limited to the following examples.

### Examples

### Preparation Example

A mascara solution was prepared according to the ingredients and contents (v/v%) as shown in the following Table 1. Herein, carnauba wax, rice bran wax and Rhus Succedanea fruit wax were used as vegetable wax ingredients.

**[Table 1]**

| Ingredients | %(v/v) | Function |
|---|---|---|
| Water | 51.6800 | Solvent |
| Acrylates Copolymer | 10.0000 | Binder |
| Iron Oxides (CI 77499) | 10.0000 | Colorant |
| Hydrogenated Coco-Glycerides | 5.0000 | Skin Conditioning Agent |
| Copernicia Cerifera (Carnauba) Wax | 4.0000 | Skin Conditioning Agent |
| Cetearyl Alcohol | 3.0000 | Emulsifier |
| Oryza Sativa (Rice) Bran Wax | 2.0000 | Skin-Conditioning Agent - Occlusive |
| Rhus Succedanea Fruit Wax | 2.0000 | Binder |
| Polyisobutylene | 2.0000 | Binder |
| Glyceryl Stearate | 2.0000 | Emulsifier |
| Hydrogenated Rapeseed Oil | 2.0000 | Skin Conditioning Agent |
| Beeswax | 1.5000 | Binder |
| Tromethamine | 1.0000 | pH Adjuster |
| Ethanol | 1.0000 | Antifoaming Agent |
| Phenoxyethanol | 0.5000 | Preservative |
| Polysorbate 60 | 0.5000 | Surfactant |
| Sorbitan Stearate | 0.5000 | Surfactant |
| Sorbitan Sesquioleate | 0.5000 | Surfactant |
| Ethylhexylglycerin | 0.4000 | Skin Conditioning Agent |
| Polyvinyl Alcohol | 0.2500 | Binder |
| Dimethicone | 0.1000 | Skin Conditioning Agent |
| Hydroxyethylcellulose | 0.0500 | Binder |
| Disodium EDTA | 0.0200 | Sequestrant |

### Test Example

The magnetic mascara tool including a mascara application head having no brush hair and provided with a magnet in the inner space of the application head was manufactured according to the present disclosure. Twenty females in their 20-40 ages were allowed to carry out eyelash makeup and the extension of eyelash and the curvature of curls were evaluated.

The curvature of curls was evaluated according to the following criteria.
- J curl: gentle curling similar to the human eyelash
- JC curl: a type of curling having a curvature between that of J curl and that of C curl to provide a natural curling effect and showing strong curling from the tail portion
- C curl: curling like those picked up by an eyelash curler
- CC curl: curling stronger than C curl

In the following Table 2, Example shows the test results of the mascara solution including iron oxide in combination with carnauba wax according to the above Preparation Example. Comparative Examples 1 to 3 show the test results of the mascara solutions obtained in the same manner as Preparation Example, except that candelilla wax (Comparative Example 1), a mixture containing carnauba wax with sunflower seed wax in the same amount (Comparative Example 2) or sugar wax (Comparative Example 3) were used instead of carnauba wax, rice bran wax and Rhus Succedanea fruit wax as vegetable wax ingredients.

**[Table 2]**

| | Example | Comp. Ex. 1 | Comp. Ex. 2 | Comp. Ex. 3 |
|---|---|---|---|---|
| Extension (average, mm) | 3.2 | 1.2 | 2.1 | 1.3 |
| Curvature of curls | JC to C curl | J curl | JC curl | JC curl |

It can be seen from the above test results that when a magnet is applied to the mascara solution according to Example provides the most significant long lash effect and forms JC curls having strong curling from the tail portion to C curls like those picked up by an eyelash curler. On the other hand, Comparative Examples 1-3 using the other vegetable wax ingredients shows lower curling and long lash effects. This is because magnetic power is applied, while carnauba wax having a depilation function draws the eyelash, and thus iron oxide attached to the end of the eyelash is linked continuously.

### [Description of Drawing Numerals]

10: Brush stick
11: Application head
11a: Application head body portion
11b: Application head terminal portion
12: Magnet
20: Container
30: Lid

## Claims

1. A mascara comprising:
a lid used as a knob;
a brush stick extended from the inner central portion of the lid; and
a container having an inner space for receiving a mascara solution,
wherein the free end of the brush stick comprises a mascara application head having no brush hair and the inner space of the application head includes a magnet.

2. The mascara according to claim 1, wherein the application head comprises a head body portion and a head terminal portion having a smaller diameter as compared to the head body portion, and the inner space of the head body portion comprises a magnet but the head terminal portion comprises no magnet.

3. The mascara according to claim 2, wherein the head body portion has a cylindrical shape and the head terminal portion is a tapered cone-shaped tip.

4. The mascara according to claim 2, wherein the head body portion has a cylindrical shape and the head terminal portion is a cylindrical tip having a smaller diameter as compared to the head body portion.

5. The mascara according to claim 1, wherein the inner space for receiving the mascara solution comprises a packing at the upper end entrance thereof, wherein the packing has an aperture-like shape.

6. The mascara according to claim 5, wherein the packing is a silicone packing.

7. The mascara according to claim 1, wherein both poles of the magnet are formed in the transverse direction of the brush stick.

8. The mascara according to claim 1, wherein the magnet is selected from the group consisting of an AlNiCo magnet, a ferrite magnet, a rare earth metal magnet, a resin magnet, a rubber magnet and a plastic magnet.

9. The mascara according to claim 1, wherein the application head is made of a metallic material.

10. The mascara according to claim 1, wherein the application head has grooves formed therein, and the grooves has a quadrangular or triangular shape.

11. The mascara according to claim 10, wherein the grooves have a depth of 0.2-0.5 mm and an interval of 0.5-0.75 mm.

12. The mascara according to claim 1, wherein the mascara solution comprises iron oxide.

13. The mascara according to claim 1, wherein the mascara solution comprises iron oxide in combination with a vegetable wax drawing the eyelash.

14. The mascara according to claim 13, wherein the vegetable wax drawing the eyelash comprises carnauba wax.

15. A mascara set comprising:
(i) one mascara brush stick; and
(ii) at least two containers including an inner space opened/closed with a lid, wherein the free end of the brush stick comprises a mascara application head having no brush hair, the inner space of the application head comprises a magnet, the containers individually comprise at least two mascara solutions having different colors, and the brush can be washed and used.
